# EUROPEAN PATENT APPLICATION

(11) **EP 4 785 868 A1**
(43) Date of publication of application: **05.08.2026**
(21) Application number: 26154825.9
(22) Date of filing: 28.01.2026
(51) Int. Cl.: A61B 6/03, A61B 6/00, A61N 5/10

(54) **SYSTEMS, APPARATUSES AND METHODS FOR MANGING IMAGING OF A PATIENT**

(30) Priority: 31.01.2025 GB 202501428
(71) Applicant: Elekta Limited, Crawley, West Sussex RH10 9BL (GB)
(72) Inventor: BAL, Matthieu, Crawley, RH10 9BL (GB); MASON, Jonathan, Crawley, RH10 9BL (GB); JOYCE, Thomas, Crawley, RH10 9BL (GB); STANCANELLO, Joseph, Crawley, RH10 9BL (GB)
(74) Representative: Haseltine Lake Kempner LLP

(57) **Abstract**

A computer-implemented method for managing Cone Beam Computed Tomography, CBCT, imaging of a patient. The method comprises acquiring (102) radiotherapy treatment plan, RTP, information for a RTP. The RTP is for delivering a radiation dose distribution to the patient so as to respect at least one radiotherapy treatment objective. The method further comprises determining (104), based on the acquired RTP information, a measure of sensitivity of the RTP to changes in anatomy of the patient. The measure of sensitivity is a function of position in the patient or a function of radiotherapy beam direction in the RTP. The method further comprises generating (106), based on the determined measure of sensitivity, an imaging protocol for obtaining a reconstructed CBCT image of a volume of the patient by setting a value of at least one parameter in the imaging protocol such that image quality of the reconstructed CBCT image of the patient volume meets an image quality objective for an image processing task. The image quality objective prioritises image quality for positions within the patient volume at which the RTP is sensitive to changes in anatomy. The imaging protocol comprises values for at least one CBCT imaging acquisition parameter and/or at least one CBCT imaging reconstruction parameter.

## Description

### Technical field

Embodiments of the present disclosure relate to systems, apparatuses and methods for managing imaging of a patient. More specifically, embodiments of the disclosure relate to a computer-implemented method for managing Cone Beam Computed Tomography imaging of a patient, and a management node, a radiotherapy apparatus and a computer program product configured to execute a method for managing Cone Beam Computed Tomography imaging of a patient.

### Background

Radiotherapy, one of the cornerstones of cancer treatment, is the use of ionising radiation to damage or destroy unhealthy cells in a human or animal body. During treatment, the ionising radiation is formed into a beam and directed to unhealthy cells in the body, such as a tumour.

Radiotherapy (RT), also referred to as radiation therapy, is performed in accordance with a radiotherapy treatment plan for delivering a radiation dose distribution to the patient to achieve a set of radiotherapy treatment objectives. The radiotherapy treatment objectives may, for example, include: a minimum radiation dose that is to be delivered to the tumour, an upper limit on radiation dose that is to be delivered to a healthy organ that is at risk of radiation damage, and an upper limit on the total radiation dose that is to be delivered to the patient. The delivered dose distribution depends on treatment parameters such as the beam direction, dose per beam energy, number of beams, beamlet weights, and exposure time. A region identified as needing treatment (e.g., a region comprising a tumour) is referred to as a clinical target volume (CTV). A region to which treatment is to be delivered is referred to as the planning target volume (PTV) and is typically the CTV plus some margin that is added to help ensure that the CTV is treated effectively. A region in which a treatment dose is undesirable is referred to as an organ at risk (OAR).

Radiotherapy treatment planning is the process of determining a radiotherapy treatment plan for delivering a suitable dose distribution that will meet the radiotherapy treatment objectives. During radiotherapy treatment planning, an anatomical region of the patient is modelled using a three-dimensional image of the patient, e.g., a computed tomography (CT) image referred to as a planning CT. Image segmentation is performed to define different regions within the image, referred to as segments, and different objectives and constraints may be defined for different segments. The result of the radiotherapy treatment planning is a radiotherapy treatment plan indicating a configuration and/or delivery sequence for a radiotherapy system for delivering a radiation dose distribution to the patient. For example, the radiotherapy treatment plan may include parameters specifying, for example, the direction, cross sectional shape, energy and intensity of each radiation beam to be applied to the patient. The radiotherapy treatment plan may include dose fractioning, in which a sequence of radiotherapy treatments is provided over a predetermined period of time, with each treatment delivering a specified fraction of the total prescribed dose. For example, the total radiation dose may be divided into around 3 to 40 daily fractions. Radiotherapy treatment planning may be performed using a treatment planning system, such as Monaco^{®}. Radiotherapy treatment planning may also be referred to as radiation treatment planning or treatment planning. A radiotherapy treatment plan may also be referred to as a radiation treatment plan or a treatment plan.

Image guided RT (IGRT) is a technique to capture the anatomy of the patient at the time of dose fraction delivery using in-room imaging, and to accurately align the planned dose distribution with the patient anatomy to increase the accuracy of the treatment. Cone Beam Computed Tomography (CBCT) is the most widely used imaging modality for IGRT. CBCT imaging of the patient for IGRT is performed in accordance with an imaging protocol. The imaging protocol may include a CBCT imaging acquisition protocol for performing a CBCT scan and/or a CBCT imaging reconstruction protocol for processing the raw data from the CBCT scan into a 3D reconstructed CBCT image.

Adaptive radiotherapy (ART) is a technique that uses imaging to evaluate and adapt the radiotherapy treatment plan. In inter-fraction ART, images obtained during a RT treatment session are analysed offline and used to adjust the radiotherapy treatment plan for future sessions (i.e., the changes are made between fractions). In intra-fraction ART, images obtained during a RT treatment session are analysed online (i.e., in real time during the session) and used to adjust the radiotherapy treatment plan for that session (i.e., the changes are made during a fraction). ART can thereby increase the accuracy of RT treatment, e.g., by ensuring that the radiotherapy treatment plan accounts for any changes in patient anatomy.

A disadvantage of IGRT and ART is that the additional dose from CBCT imaging performed during treatment (referred to as the imaging dose) can increase the risk of secondary cancers. Further information can be found in Alaei, P., & Spezi, E., 2015, Phys. Med. Vol 31 No. 7. To minimise the risk of secondary cancers, it is important to keep the CBCT imaging dose during radiotherapy treatment as low as possible. It is also advantageous to keep the CBCT acquisition and reconstruction time as short as possible, e.g., for patient comfort, for treatment of a larger number of patients in a given time, and for tracking faster patient movements, e.g. for ART. However, minimising the imaging dose and imaging time must be balanced with the CBCT image quality (e.g., signal to noise ratio and/or image resolution of the CBCT image). The image quality is influenced by parameters in the CBCT acquisition protocol such as the projection angles of the CBCT projections (e.g., density of projection angles along a scan arc), the gantry speed for the CBCT projections, and the intensity (mAs) and energy (kV) of the imaging source. For example, increasing the source intensity (by increasing the X-ray tube current (mA) and/or exposure time (s)) increases the signal-to-noise ratio of the acquired images. Increasing the source energy improves penetration of the X-rays, which may improve image quality by increasing the signal to noise ratio at the detector, however increasing the source energy may also decrease image contrast by reducing the difference in absorption (i.e., the absorption differential) for different tissues. The impact of imaging protocol parameters on the image quality has been studied, for example, in Xuanhao, Z et al., 2022 Measurement, 194, 111061; and Yan, H et al., 2012 Phys. Med. Biol. 57 2063. The image quality is also influenced by parameters in the CBCT reconstruction protocol such as voxel size and the number of iterations in the reconstruction algorithm.

In radiotherapy applications, sufficiently high image quality is important for accurately performing image processing tasks such as imaging contouring and image segmentation, which are key components of radiotherapy treatment planning. The need for high quality CT images for radiotherapy treatment planning is described in Chen, G-P et al., 2017 Physics and Imaging in Radiation Oncology 4 (6-11).

CBCT image quality may be maximised for radiotherapy guidance by generating the CBCT imaging protocol based on body mass index (BMI) or water equivalent thickness (WET) of the patient. When generating the imaging protocol, parameters such as the source intensity, source energy, exposure time, and/or detector gain may be chosen to achieve the desired penetration, contrast and/or artifact reduction. For example, the radiological depth of patient tissue (expressed as a WET) can be used to determine the imaging intensity and exposure time that is needed to penetrate the tissue for a specified minimum image quality. Denser tissue or thicker regions of the patient will require higher intensity and longer exposure times. Similarly, the BMI of the patient may indicate that a higher or lower source intensity and exposure time should be used to achieve sufficient image contrast with minimal radiation dose.

### Summary

High quality CBCT images are important for RT applications such as radiotherapy treatment planning, IGRT, and ART since the CBCT image quality influences the accuracy of subsequent image processing task(s) such as image reconstruction, image contouring and image segmentation. The accuracy of the image processing task(s) in turn impacts the accuracy of subsequent RT treatment. For example, image segmentation is commonly performed on a reconstructed CBCT image to identify the shape and position of the patient anatomy at the time of treatment delivery (e.g., for IGRT and/or ART), and therefore it is important that the image quality of the CBCT images acquired during treatment is sufficient for accurate organ delineation and dose calculation. Therefore, there is a need to balance the requirement to minimise the CBCT imaging dose and/or imaging time (for acquisition and/or reconstruction) with the requirement to acquire a reconstructed CBCT image that is of sufficiently high quality for subsequent image processing task(s).

According to a first aspect of the present disclosure, there is provided a computer-implemented method for managing CBCT imaging of a patient. The method comprises acquiring radiotherapy treatment plan information for a radiotherapy treatment plan (RTP). The RTP is for delivering a radiation dose distribution to the patient so as to respect at least one radiotherapy treatment objective. The method further comprises determining, based on the acquired RTP information, a measure of sensitivity of the RTP to changes in anatomy of the patient. The measure of sensitivity is a function of position in the patient or a function of radiotherapy beam direction in the RTP. The method further comprises generating, based on the determined measure of sensitivity, an imaging protocol for obtaining a reconstructed CBCT image of a volume of the patient. The imaging protocol comprises values for at least one CBCT imaging acquisition parameter and/or at least one CBCT imaging reconstruction parameter. The imaging protocol is generated by setting a value of at least one parameter in the imaging protocol such that image quality of the reconstructed CBCT image of the patient volume meets an image quality objective for an image processing task. The image quality objective prioritises image quality for positions within the patient volume at which the RTP is sensitive to changes in anatomy.

According to another aspect of the present disclosure, there is provided a management node for managing CBCT imaging of a patient. The management node comprises processing circuitry configured to cause the management node to acquire RTP information for a RTP. The RTP is for delivering a radiation dose distribution to the patient so as to respect at least one radiotherapy treatment objective. The management node is further configured to determine, based on the acquired RTP information, a measure of sensitivity of the RTP to changes in anatomy of the patient. The measure of sensitivity is a function of position in the patient or a function of radiotherapy beam direction in the RTP. The management node is further configured to generate, based on the determined measure of sensitivity, an imaging protocol for obtaining a reconstructed CBCT image of a volume of the patient by setting a value of at least one parameter in the imaging protocol such that image quality of the reconstructed CBCT image of the patient volume meets an image quality objective for an image processing task. The image quality objective prioritises image quality for positions within the patient volume at which the RTP is sensitive to changes in anatomy. The imaging protocol comprises values for at least one CBCT imaging acquisition parameter and/or at least one CBCT imaging reconstruction parameter.

According to another aspect of the present disclosure, there is provided a computer program product comprising a computer readable medium, the computer readable medium having computer readable code embodied therein, the computer readable code being configured such that, on execution by a suitable computer or processor, the computer or processor is caused to perform a method according to any one or more aspects or examples of the present disclosure.

According to another aspect of the present disclosure, there is provided a radiotherapy treatment apparatus comprising a management node according to the present disclosure.

Thus, the present disclosure provides improved methods, apparatuses and systems for managing CBCT imaging of a patient. By prioritising the image quality at positions within the patient volume at which the RTP is sensitive to changes in patient anatomy, the imaging dose delivered to the patient, and/or the imaging time for image acquisition and/or reconstruction, may be minimised whilst obtaining a reconstructed CBCT image that meets the necessary image quality requirements for a given image processing task. The present techniques thereby ensure the accuracy of RT treatment tasks that may be performed based on the reconstructed CBCT image.

Embodiments of the disclosure may be implemented in digital electronic circuitry, or in computer hardware, firmware, software, or in combinations thereof. Embodiments of the disclosure may be implemented as a computer program or a computer program product, i.e., a computer program tangibly embodied in a non-transitory information carrier, e.g., in a machine-readable storage device or in a propagated signal, for execution by, or to control the operation of, one or more hardware modules. A computer program may be in the form of a stand-alone program, a computer program portion, or more than one computer program, and may be written in any form of programming language, including compiled or interpreted languages, and it may be deployed in any form, including as a stand-alone program or as a module, component, subroutine, or other unit suitable for use in a data processing environment.

The disclosure is set out herein in terms of particular embodiments. Other embodiments, not explicitly described here, may nonetheless fall within the scope of the claims. Unless explicitly or implicitly specified otherwise, the steps of methods according to embodiments of the disclosure may be performed in a different order and still achieve desirable results.

### Brief description of the drawings

Exemplary embodiments will now be described, by way of example only, with reference to the following drawings, in which:
Figure 1 is a flow chart of a computer-implemented method for managing CBCT imaging of a patient;
Figure 2 is a series of images illustrating an example method for managing CBCT imaging of a patient;
Figure 3 is a diagram of the geometric arrangement for cone beam projection scanning and reconstruction;
Figure 4 is a diagram of a cone beam CT scanner;
Figure 5 is a block diagram illustrating a management node for managing CBCT imaging of a patient;
Figure 6 is a schematic illustration of a radiotherapy apparatus; and
Figure 7 is a block diagram illustrating a radiotherapy system suitable for managing CBCT imaging of a patient.

### Detailed description of embodiments

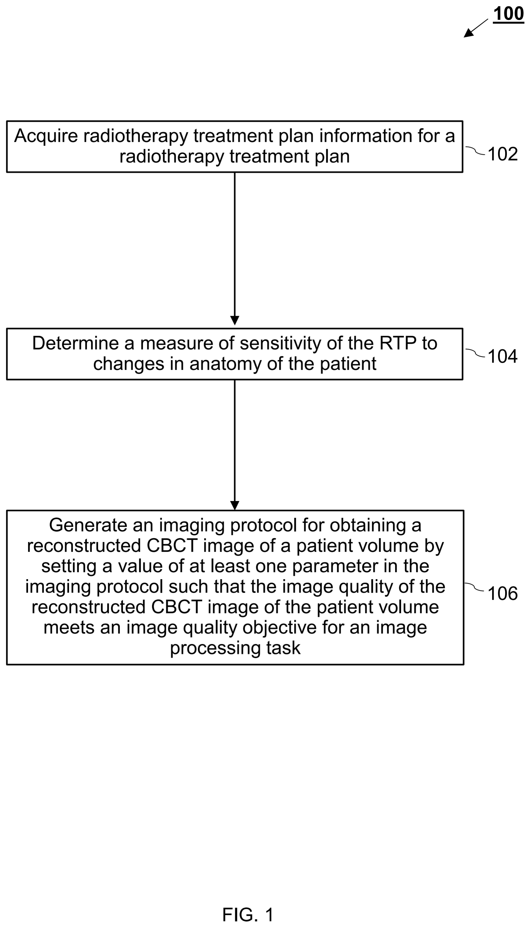

Various aspects and details of the present disclosure are now described with reference to Figures 1 to 7.

**Figure 1** is a flow chart showing a computer-implemented method 100 for managing CBCT imaging of a patient according to the present disclosure. The method 100 addresses the need to acquire images that are of a sufficiently high quality for image processing task(s) whilst minimising the imaging dose delivered to the patient and/or the imaging acquisition and/or reconstruction time. The method 100 may be for use in image guided radiotherapy and/or adaptive radiotherapy.

At step 102, the method 100 comprises acquiring radiotherapy treatment plan information for a radiotherapy treatment plan. The RTP is for delivering a radiation dose distribution to the patient so as to respect at least one radiotherapy treatment objective. Radiotherapy treatment plan information may refer to information generated during a radiotherapy treatment planning process for the patient (in which the RTP was generated). The RTP information may comprise one or more of: the RTP for the patient; the radiation dose distribution for the patient; a gradient of the radiation dose distribution for the patient (also referred to as a dose gradient distribution or dose gradient map); a radiation fluence map; a Dose Volume Histogram (DVH) for the patient; radiation to be delivered to the patient as a function of radiotherapy beam direction in the RTP; monitor units (MU) as a function of radiotherapy beam direction (e.g., gantry angle or control point for the radiotherapy beam) in the RTP; an image of the patient (e.g., a CT image or MRI image); a segmented image of the patient (e.g., including the target volume and one or more OARs); a patient model (e.g., a 3D patient model); a configuration of a radiotherapy system for delivering the radiation dose distribution; one or more parameters for configuring the radiotherapy system (e.g., one or more of: RT beamlets weights, RT beam angles, dose-histogram-volume information, a number of RT beams, dose per RT beam, isocenter, and rotation plane); and a delivery sequence for the radiotherapy system for delivering the radiation dose distribution.

The RTP information may be acquired by receiving the RTP information from another entity or by retrieving the RTP information from computer memory. Alternatively, acquiring the RTP information may comprise generating the RTP information, e.g., by performing a radiotherapy treatment planning procedure; performing an optimization procedure; using a machine learning model; and/or performing other methods for generating the radiotherapy treatment plan information that are known in the art.

At step 104, the method 100 comprises determining, based on the acquired radiotherapy treatment plan information, a measure of sensitivity of the RTP to changes in anatomy of the patient. The measure of sensitivity may be a function of position (e.g., voxel) in the patient. For example, the measure of sensitivity may be a function of position (i.e., location, e.g., voxel position/location) in an image of the patient (where the image may be comprised in the RTP information). Alternatively, the measure of sensitivity may be a function of radiotherapy beam direction in the RTP.

The measure of sensitivity may represent a degree to which the RTP would require adaptation as a consequence of changes to the patient anatomy in order to respect one or more of the at least one radiotherapy treatment objective. For example, the measure of sensitivity as a function of position in the patient may represent a degree to which the RTP would require adaptation as a consequence of changes to the patient anatomy at a plurality of positions in the patient. As another example, the measure of sensitivity as a function of radiotherapy beam direction in the RTP may represent a sensitivity of the RTP for a plurality of RT beam directions in the RTP (e.g., for a plurality of gantry angles or control points in the RTP) to changes in the patient anatomy.

The measure of sensitivity may comprise one or more of: a gradient of the radiation dose distribution as a function of position in the patient; a scale of patient anatomy change that would necessitate adaptation of the RTP in order to respect one or more of the at least one radiotherapy treatment objective as a function of position in the patient; the radiation dose distribution as a function of position in the patient; radiation to be delivered to the patient as a function of radiotherapy beam direction in the RTP; and monitor units to be delivered as a function of radiotherapy beam direction according to the RTP (i.e., planned amount of radiation/monitor units to be delivered according to the RTP for the patient). Monitor units are a measure of the amount of radiation to be delivered. As used herein, a dose gradient distribution may comprise a dose gradient vector field.

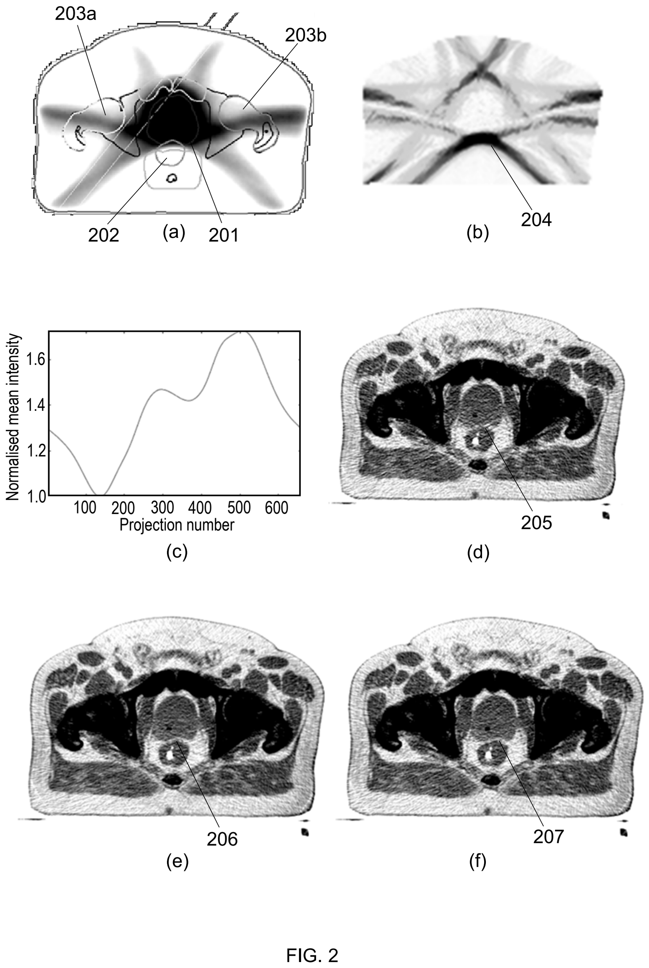

A large dose gradient is indicative of sensitivity of the RTP to changes in anatomy because it indicates that at that position in the patient there is a large change in dose requirements. For example, the position may correspond to a boundary between a PTV and an OAR. The radiotherapy treatment plan will be sensitive to changes in the anatomy of the patient at this position because movement of the OAR into the expected position of the PTV will result in the OAR receiving a significantly higher radiation dose than planned, which could violate a radiotherapy treatment objective. Similarly, if a section of the PTV moves into the expected position of the OAR, then the PTV will receive a lower radiation dose than planned, which could again violate a radiotherapy treatment objective. Higher image resolution is therefore required at this position (see step 106), e.g., to accurately delineate the regions of differing dose requirements. An example of the method 100 of Figure 1 in which the measure of sensitivity comprises a dose gradient distribution is described with reference to Figure 2.

The measure of sensitivity may comprise both a dose gradient distribution and the radiation dose distribution. Including the radiation dose distribution in the measure of sensitivity helps in identifying the positions in the patient at which the radiotherapy treatment plan is sensitive to anatomy changes because if a given position has high dose gradient but low dose, it is unlikely that changes in the patient anatomy at this position will violate any of the RT treatment objectives. For example, a RT treatment objective of the RTP may require that the dose delivered to an OAR does not exceed a threshold value. If the dose in the given position mentioned above is significantly below this threshold dose value, then the RT treatment objective will not be violated by changes in patient anatomy at this position. Thus, image quality need not be prioritised at these positions (see step 106 below).

A large value for the planned amount of radiation (e.g., monitor units) to be delivered to the patient for a given RT beam direction is indicative of sensitivity of the RTP to changes in anatomy because it indicates that, for that RT beam direction, the RTP is sensitive to changes in anatomy. This is because if a high dose value (e.g., high MU) is intended for a given RT beam direction, then the radiation delivered in this RT beam direction is more likely to violate a RT treatment objective due to a change in patient anatomy than the radiation delivered in a different RT beam direction for which a lower dose value (lower MU) is intended. For example, if the large dose delivered in this RT beam direction is intended for a first position in the patient (e.g., the tumour) but is instead delivered to a second position in the patient due to a change in the patient anatomy (e.g., due to anatomical movement), then a radiotherapy treatment objective may be violated (e.g., because the first position receives too low a dose for effective treatment of the tumour, and/or the second position corresponds to healthy tissue which therefore receives too high a dose). Thus, it is beneficial to prioritise image quality for CBCT image acquisition corresponding to the RT beam directions with higher MU (see step 106).

The changes in anatomy of the patient may comprise (be a result of) one or more of: soft tissue movement; soft tissue structure shape changes; and overall patient anatomy changes. For example, an organ or other soft tissue movement may comprise cyclic movement such as breathing or cardiac movement. An organ or other soft tissue movement may additionally or alternatively comprise a transitory movement, e.g., as a result of transient gas bubbles. Soft tissue structure shape changes may comprise changes to an organ and/or a target volume for the radiotherapy treatment such as a tumour. Changes in overall patient anatomy may comprise cumulative changes such as those resulting from weight loss, weight gain, muscle building, muscle loss, surgery and/or injury.

Determining the measure of sensitivity of the RTP to changes in anatomy of the patient may comprise performing at least one of: an analytic process; an optimization process; and/or a Machine Learning (ML) process. Using an ML process to determine a measure of sensitivity of the RTP to changes in anatomy of the patient may comprise inputting to an ML model the radiotherapy treatment plan information. The ML model may be operable to output the measure of sensitivity.

Determining the measure of sensitivity of the RTP to changes in the anatomy of the patient may comprise: obtaining a prediction of patient movement during the CBCT imaging of the patient and/or during the radiotherapy treatment of the patient; and evaluating sensitivity of the RTP to the predicted patient movement. For example, it may be determined that a position within the patient is sensitive if the predicted movement at that position would cause the violation of at least one RT treatment objective. The prediction of patient movement may be obtained based on measurements of patient movement (e.g., Michael J. Zelefsky, et al., Radiotherapy and Oncology, Volume 50, Issue 2, 1999, Pages 225-234). The prediction of patient movement may be obtained using a deep learning model (e.g., Oscar Pastor-Serrano et al. 2023 Phys. Med. Biol. 68 085018).

Performing an analytic process to determine 104 a measure of sensitivity may comprise the use of other treatment planning tools such as plan robustness and plan sensitivity analysis.

Results from any of the analytic approaches to step 104 mentioned herein could be used to train an Al model to perform step 104. In further embodiments, an optimization process could be used to determine a measure of sensitivity. Examples of such methods are provided in Unkelbach J, et al., Phys Med Biol. 2018 Nov 12; 63 (22), 30418942.

At step 106, the method 100 comprises generating, based on the determined measure of sensitivity, an imaging protocol for obtaining a reconstructed CBCT image of a volume of the patient (i.e., of a patient volume). The imaging protocol comprises values for at least one CBCT imaging acquisition parameter and/or at least one CBCT imaging reconstruction parameter. Thus, the imaging protocol may be an image acquisition protocol for acquiring CBCT projections of the patient (for use in subsequent image reconstruction) and/or an image reconstruction protocol for performing image reconstruction of acquired CBCT projections to obtain a reconstructed CBCT image of the patient.

The imaging protocol is generated in step 106 by setting a value of at least one parameter in the imaging protocol such that image quality of the reconstructed CBCT image of the patient meets an image quality objective for an image processing task. The image quality objective prioritises image quality for positions within the patient volume at which the RTP is sensitive to changes in anatomy. The image quality objective may prioritise image quality for positions within the patient volume at which the RTP is sensitive to changes in anatomy relative to positions within the patient volume at which the RTP is less sensitive to changes in anatomy. The image quality objective may be based on (e.g., expressed in terms of) the determined measure of sensitivity. Image quality of the reconstructed CBCT image may comprise (e.g., be measured in terms of) one or more of: signal to noise ratio (SNR), noise, spatial resolution, contrast resolution, contrast to noise ratio (CNR), Hounsfield fidelity, image uniformity, and geometrical precision.

Prioritising image quality for positions within the patient volume at which the RTP is sensitive to changes in anatomy is beneficial because these are the positions at which the accuracy of the image processing task is most important for RT applications such as accurate RT delivery. For example, these are the positions at which the accuracy of the image processing task (e.g., image reconstruction, contouring, segmentation, deformation, registration) is most important for meeting the RT treatment objectives of the RTP.

The at least one CBCT imaging acquisition parameter may comprise one or more of: imaging radiation source location; imaging detector location; imaging projection angle; imaging radiation source intensity; source (e.g., X-ray) tube current for one or more projections; exposure time for one or more projections; imaging radiation source energy for one or more projections; number of projections per gantry angular range; gantry rotation speed for one or more projections; gantry angular range; rotation plane; isocentre; image projection angle for one or more projections; imaging beam shape for one or more projections; detector offset for one or more projections; detector sensitivity for one or more projections; and detector gain for one or more projections. Any one or more of these parameters may vary for different projections across a CBCT scan (i.e., any of these parameters may be defined for one or more projections or may be defined as a function of projection angle/number/position). Generating the imaging protocol may comprise determining values for at least one of these CBCT imaging acquisition parameters as a function of projection angle in the imaging protocol.

The at least one CBCT imaging reconstruction parameter may comprise one or more of: voxel size for one or more regions of the patient volume; number of voxels for one or more regions of the reconstructed CBCT image; voxel density for one or more regions of the reconstructed CBCT image; voxel distribution for one or more regions of the reconstructed CBCT image; one or more regularisation terms (in the reconstruction algorithm); number of iterations (in the reconstruction algorithm); and a stopping criterion (in the reconstruction algorithm). Any one or more of these parameters may vary for different positions across the imaged volume (i.e., any of these parameters may be defined for one or more voxels or may be defined as a function of position in the reconstructed CBCT image). Generating the imaging protocol may comprise determining values for at least one of these CBCT imaging reconstruction parameters as a function of position in the reconstructed CBCT image of the patient.

Prioritising image quality for positions within the patient volume at which the RTP is sensitive to changes in anatomy may be achieved by increasing image quality at sensitive positions according to the determined measure of sensitivity, e.g., by increasing voxel density at more sensitive positions relative to the voxel density at less sensitive positions. Alternatively, prioritising image quality for positions within the patient volume at which the RTP is sensitive to changes in anatomy may be achieved indirectly, e.g., by increasing image quality for certain CBCT projection angles such as projection angles with a higher measure of sensitivity relative to projection angles with a lower measure of sensitivity. For example, the imaging source intensity and/or imaging source energy may be increased for CBCT projections with a higher measure of sensitivity relative to the imaging source intensity and/or imaging source energy for CBCT projections with a lower measure of sensitivity. Both direct and indirect approaches to prioritising image quality for positions within the patient volume at which the RTP is sensitive to changes in anatomy are described in more detail below.

The image processing task comprises an image processing task to be performed on the reconstructed CBCT image of the patient. The image processing task may comprise one or more of: image reconstruction, image segmentation, image contouring; identification of a target region; identification of an organ at risk; identification of an isocentre; identification of an isocentre shift (e.g., due to patient movement); image deformation; and/or image registration (e.g., for patient tissue motion tracking). The confidence associated with an output of the image processing task may be influenced by image quality at positions within the patient volume at which the RTP is sensitive to changes in anatomy.

The image quality objective may seek to balance a first requirement to optimize the image quality of the reconstructed CBCT image for the image processing task, with one or both of: a second requirement to minimise total imaging radiation dose delivered to the patient during CBCT image acquisition, and a third requirement to minimise total computation time for image reconstruction. This balance may be achieved by prioritising image quality for (i) positions within the patient volume at which the RTP is sensitive to changes in anatomy, and/or (ii) projections within a CBCT scan for which the RTP is sensitive to changes in anatomy. For example, the image quality objective may drive an increase in image quality of the reconstructed CBCT image for certain positions within the patient volume and/or projection angles within a CBCT scan so as to improve the quality and/or robustness of the image processing task, but not so much of an increase that the patient receives an unnecessarily high imaging dose and/or the computation time is unnecessarily high. Thus, the image quality objective may motivate a decrease in image quality of the reconstructed CBCT image for positions within the patient volume and/or projections within a CBCT scan for which the RTP is less sensitive to changes in anatomy. Minimising computation time is particularly advantageous for real-time ART since patient motion can occur quickly (i.e., over a short period of time). This could include motion due to breathing, digestion, blood circulation, sneezing, twitching, etc. Reducing computation time enables faster motions to be tracked and accounted for in RT treatment plan adaptations for more accurate treatment delivery.

The image quality objective may comprise a performance criterion for the image processing task performed on the reconstructed CBCT image. Thus, the image quality of the reconstructed CBCT image of the patient meeting the image quality objective may comprise the image processing task satisfying the performance criterion when performed on the reconstructed CBCT image of the patient. For example, for image segmentation, the performance criterion may comprise a maximum uncertainty level for a segment boundary or contour. Thus, the image quality objective may drive an increase in image quality for positions within the patient volume at which the segmentation method struggles to identify a segment boundary (e.g., an organ outline). For example, in model-based segmentation, the uncertainty level may comprise a threshold external energy value, e.g., the performance criterion may comprise a requirement that the external energy is below the threshold external energy value. Alternatively, determining whether the image processing task satisfies a performance criterion may comprise deep learning methods. An example for image segmentation is provided by Mehrtash A, at al., IEEE Trans Med Imaging. 2020 Dec;39(12):3868-3878.

The image quality objective may be determined based on a planning CT for the patient, or it may be predefined.

Positions within the patient volume at which the RTP is sensitive to changes in anatomy may comprise positions and/or projection angles for which the measure of sensitivity satisfies a sensitivity criterion. The sensitivity criterion may comprise one or more of: a threshold radiation dose gradient; a reference scale anatomy change that would necessitate adaptation of the RTP in order to respect one or more of the at least one RT treatment objective; a threshold level of radiation to be delivered to the patient; and a threshold level of MUs to be delivered to the patient.

For example, the sensitivity criterion may comprise a threshold dose gradient value, and a position that satisfies the sensitivity criterion may comprise a position at which the gradient of the radiation dose distribution is above the threshold dose gradient value. Positions at which higher quality imaging is required are characterized by steep dose gradients because it is an indication that segments of the patient with different requirements (e.g., a segment comprising the tumour versus a segment comprising an OAR) are relatively close to each other. The sensitivity criterion may exclude dose gradient values at the skin-air interfaces where the dose gradient is expected to be high. This is because it is not usually necessary to obtain high quality images of the skin-air interface since the external body contour (which is used for dose calculation) can be acquired with much less radiation than for internal contours.

In examples where the measure of sensitivity comprises a dose gradient distribution, the radiotherapy treatment plan information may comprise the radiation dose distribution for the patient, and determining the measure of sensitivity may comprise calculating, based on the radiation dose distribution, a gradient of the radiation dose distribution as a function of position in the patient. The radiation dose distribution may have been generated during radiotherapy treatment planning for the patient. Thus, the radiation dose distribution may be determined based on the radiotherapy treatment plan, or it may have been stored during radiotherapy treatment planning.

Setting a value of at least one parameter in the imaging protocol such that the image quality objective is met may comprise selecting value(s) for at least one CBCT imaging acquisition parameter that is different for projections for which the sensitivity criterion is met compared to value(s) of the at least one CBCT imaging acquisition parameter for projections for which the sensitivity criterion is not met. For example, a higher imaging intensity; a slower gantry speed; and/or a smaller angular spacing between projections may be selected for projections for which the sensitivity criterion is met. There may be a step change in the at least one CBCT imaging acquisition parameter value for certain projections. Alternatively, the at least one CBCT imaging acquisition parameter value may vary more gradually for different projections (e.g., a continuous function of CBCT projection direction/angle). In some embodiments, setting the value of the at least one CBCT imaging acquisition parameter may comprise applying a modulation function (also referred to as a modulation signal) to a set of reference values for the at least one CBCT imaging acquisition parameter, e.g., based on a reference imaging protocol. The modulation function may be chosen such that the image quality objective is met. In some examples, the radiological depth may be used to determine the parameter value(s) that are required to obtain a specified image quality (signal to noise ratio). This is advantageous for minimising imaging dose during image acquisition.

Setting a value of at least one parameter in the imaging protocol such that the image quality objective is met may comprise selecting value(s) for at least one CBCT imaging reconstruction parameter that varies as a function of position (e.g., voxel) in the patient. For example, the at least one CBCT imaging reconstruction parameters may have different value(s) for positions (e.g., voxels) in the patient for which the sensitivity criterion is met compared to the value(s) of the at least one CBCT imaging reconstruction parameter for positions (e.g., voxels) for which the sensitivity criterion is not met. For example, the at least one CBCT imaging reconstruction parameter may comprise voxel size, and a smaller voxel size may be selected for positions/voxels for which the sensitivity criterion is met compared to a voxel size selected for positions/voxels for which the sensitivity criterion is not met. Thus, the voxel sizes may be set such that the image quality of the reconstructed CBCT image meets the image quality objective for the image processing task. This is advantageous for reducing the computational time of image reconstruction, e.g., during real-time ART.

Setting a value of at least one parameter in the imaging protocol may comprise setting values for the CBCT imaging acquisition parameter(s) and the CBCT imaging reconstruction parameter(s) together to achieve a protocol that best satisfies the image quality objective.

Setting a value of at least one parameter in the imaging protocol such that the image quality objective is met may comprise selecting a parameter in the imaging protocol; and setting a value for that parameter. In some embodiments, a combination of parameters in the imaging protocol may be selected, and the values of each of these parameters may be set to achieve the image quality objective. The at least one parameter may be selected based on a finding that the parameter (or combination of parameters) is well suited to adaptation to meet the image quality objective.

Setting the value of the at least one parameter may comprise determining a relationship between the at least one parameter and the image quality of the reconstructed image. For example, the image quality may be determined for different imaging protocols (and thus different values of the at least one parameter in the imaging protocol). The image quality may be determined using an analytical function relating image quality to the imaging protocol (e.g., to imaging acquisition parameter(s) and/or to imaging reconstruction parameter(s)). It will be appreciated by the skilled person that the relationship between the parameters of an imaging protocol and the image quality of a reconstructed image obtained using the imaging protocol is analytically derivable. Alternatively, the image quality may be predicted for a given imaging protocol using one or more ML models. Use of ML in this manner may, in some instances, improve processing efficiency and accuracy. The methods for relating image acquisition parameters to a specific position/region of interest may be based on known methods used for obtaining scout scans for magnetic resonance (MR) and CT acquisitions.

Setting the value of the at least one parameter may comprise setting a value based on a level of influence that the at least one parameter has in determining the image quality of the reconstructed CBCT image for positions within the patient volume at which the RTP is sensitive to changes in anatomy. For example, parameters that have a large influence in determining the image quality of the reconstructed CBCT image for positions within the patient volume at which the RTP is sensitive to changes in anatomy may be set to different values to parameters that have a smaller influence.

Step 106 of generating an imaging protocol for obtaining a reconstructed CBCT image of the patient volume by setting a value of at least one parameter in the imaging protocol may comprise performing one or more of: an analytic process; an optimisation procedure; and/or a ML process, as discussed below.

Step 106 of generating an imaging protocol for the patient may comprise generating a new imaging protocol. A new imaging protocol may be generated by mapping the measure of sensitivity to parameters of the imaging protocol. A new imaging protocol may be generated based on a planning CT structure and the dose distribution to be delivered, e.g., by inputting the planning CT structure and the dose distribution into a ML model.

Setting a value of at least one parameter in the imaging protocol may comprise mapping the measure of sensitivity to the at least one parameter. For example, mapping the measure of sensitivity into the at least one parameter may comprise applying a scaling factor. The scaling factor may be determined based on the image quality objective (e.g., such that the image quality objective is met).

Setting a value of at least one parameter in the imaging protocol may comprise: determining a modulation function based on the measure of sensitivity; and applying the modulation function to the at least one parameter (e.g., in a reference imaging protocol) to obtain the imaging protocol. The modulation function may be scaled such that the image quality objective is met. For example, determining the modulation function may comprise applying a scaling factor. The scaling factor may be determined such that total reconstruction time for the imaging protocol is below a threshold total reconstruction time and/or such that total imaging dose for the imaging protocol is below a threshold total imaging dose. An example of scaling the modulation function in this way is described with reference to Figure 2(c). Examples of determining a modulation function based on the measure of sensitivity are now described.

For example, setting the value of at least one parameter in the imaging protocol may comprise projecting the determined measure of sensitivity (where the determined measure of sensitivity is a function of position in the patient) according to a geometry of the CBCT imaging of the patient to obtain a projected measure of sensitivity as a function of CBCT projection angle. The determined measure of sensitivity as a function of position may comprise a vector field (such as the dose gradient distribution). Projecting the determined measure of sensitivity according to the CBCT imaging geometry may comprise projecting the vector field onto a plane of the CBCT detector as a function of projection angle along the CBCT scan arc. Projecting the measure of sensitivity according to the CBCT imaging geometry may further comprise obtaining the magnitude of the in-plane component and optionally averaging over the detector plane. Thus, the projected measure of sensitivity may comprise a mean projected value for the sensitivity measure as a function of CBCT projection angle.

Setting the value of at least one parameter in the imaging protocol may further comprise determining a value for at least one CBCT imaging acquisition parameter as a function of CBCT projection angle based on the projected measure of sensitivity and the image quality objective. Determining the value for at least one CBCT imaging acquisition parameter may comprise applying a scaling factor to the projected measure of sensitivity such that the image quality of the reconstructed CBCT image of the patient meets the image quality objective for the image processing task. For example, the at least one CBCT imaging acquisition parameter as a function of CBCT projection angle may be determined by applying a scaling factor to the mean projected value for the sensitivity measure as a function of CBCT projection angle. A value for the scaling factor may be determined such that the image quality of the reconstructed CBCT image of the patient (according to the imaging protocol) meets the image quality objective for the image processing task. The CBCT imaging acquisition parameter may be, for example, the CBCT imaging intensity. Modulating the CBCT imaging intensity based on the in-plane projected measure of sensitivity in this way will result in higher intensity being used for projections in which the component of the dose gradient orthogonal to the beam axis is large. This results in higher beam intensity being used for projections that image sensitive regions such as boundaries between high dose and low dose.

The determined measure of sensitivity may comprise the radiation dose gradient as a function of position in the patient. According to this example, the radiotherapy treatment plan information acquired in step 102 may comprise the radiation dose distribution for the patient (generated during the radiotherapy treatment planning process). The radiation dose distribution may be used to determine (in step 104) a dose gradient distribution, which corresponds to a measure of sensitivity of the RTP to changes in anatomy as a function of position in the patient. Generating the imaging protocol in step 106 may comprise projecting the dose gradient distribution according to the CBCT imaging geometry to obtain a projected dose gradient (an example method of generating a projected dose gradient is described in more detail with reference to Figure 2); and determining a value of the at least one CBCT imaging acquisition parameter such that the image quality of the reconstructed CBCT image of the patient meets the image quality objective for the image processing task. Determining the value of the at least one CBCT imaging acquisition parameter may comprise normalising the projected dose gradient and modulating the at least one CBCT imaging acquisition parameter as a function of CBCT projection angle using the normalised projected dose gradient. For example, the normalised projected dose gradient may be used to modulate the imaging source intensity as a function of projection angle (such that a higher intensity beam is used for projections in which the component of the dose gradient that is orthogonal to the beam axis is large, e.g., projections in which the dose gradient is orthogonal to the beam axis).

In another example, projecting the determined measure of sensitivity according to the CBCT imaging geometry may comprise projecting the vector field perpendicular to the detector plane as a function of projection angle along the CBCT scan arc. Projecting the determined measure of sensitivity according to the CBCT imaging geometry may further comprise obtaining the magnitude of the out-of-plane component of the sensitivity measure and optionally averaging this component over the detector plane to obtain a mean out-of-plane projected value for the sensitivity measure as a function of CBCT projection angle. Thus, the projected measure of sensitivity may comprise a mean out-of-plane projected value for the sensitivity measure as a function of CBCT projection angle. Setting the value of at least one parameter in the imaging protocol may comprise determining a value for at least one CBCT imaging acquisition parameter as a function of CBCT projection angle based on the projected measure of sensitivity and the image quality objective. Determining the value for at least one CBCT imaging acquisition parameter may comprise applying a scaling factor to the projected measure of sensitivity such that the image quality of the reconstructed CBCT image of the patient meets the image quality objective for the image processing task. A value for the scaling factor may be determined such that the image quality of the reconstructed CBCT image of the patient (according to the imaging protocol) meets the image quality objective for the image processing task. The CBCT imaging acquisition parameter may be, for example, the density of CBCT projection angles along the CBCT scan arc. Modulating the CBCT angular density based on the out-of-plane projected measure of sensitivity in this way will result in a higher density of projections being acquired where the projections are moving through sensitive regions such as boundaries between high dose and low dose. The CBCT imaging acquisition parameter may alternatively be the gantry speed along the CBCT scan and values for the gantry speed may be determined based on the out-of-plane projected measure of sensitivity such that the speed is slower when out-of-plane projected measure of sensitivity is higher (i.e., an inverse relationship). This results in a slower gantry speed being used when the projections are moving through sensitive regions.

In another example, setting a value of at least one parameter in the imaging protocol may comprise determining a value for at least one CBCT imaging reconstruction parameter as a function of position in the patient volume based on the determined measure of sensitivity (where the determined measure of sensitivity is a function of position across the patient volume) and the image quality objective. Determining the value for at least one CBCT imaging reconstruction parameter may comprise applying a scaling factor to the determined measure of sensitivity such that the image quality of the reconstructed CBCT image of the patient meets the image quality objective for the image processing task. For example, the determined measure of sensitivity may comprise the radiation dose gradient as a function of position in the patient volume, and the at least one CBCT imaging reconstruction parameter may comprise voxel density in the reconstructed image. Thus, the dose gradient could be used to modulate the voxel density as a function of position in the reconstructed image (such that a higher voxel density is used for reconstructing positions within the patient volume at which the RTP is sensitive to changes in anatomy).

In another example, setting a value of at least one parameter in the imaging protocol may comprise determining a value for at least one CBCT imaging reconstruction parameter as a function of CBCT projection angle based on the determined measure of sensitivity (where the determined measure of sensitivity is a function of RT beam direction in the RTP) and the image quality objective. For example, the MU per control point / gantry angle of the RTP (e.g., for VMAT delivery) may be mapped to the intensity of the CBCT projections in the imaging protocol (as a function of CBCT projection angle).

As a further example, the sensitivity measure (e.g., the normalised projected dose gradient) could be used to modulate the number of iterations in the reconstruction algorithm (such that a larger number of iterations are used when reconstructing projections for which the RTP is sensitive to changes in anatomy such that image quality is prioritised for these projections).

In each example, the mapping of the sensitivity measure to values for the at least one parameter may be determined such that the image quality of the reconstructed CBCT image of the patient meets the image quality objective for the image processing task.

Step 106 of generating an imaging protocol may comprise generating a plurality of candidate imaging protocols for the patient; and selecting one of the plurality of candidate imaging protocols for implementation. One of the plurality of candidate imaging protocols may be selected according to a selection criterion comprising at least one of: total imaging radiation dose according to the candidate imaging protocols; image quality of reconstructed image according to the candidate imaging protocols; total imaging and/or reconstruction time according to the candidate imaging protocols; and an extent to which the image quality objective is met by the candidate imaging protocols. The selection criterion may comprise a plurality of criteria and a priority ranking. For example, if two of the candidate imaging protocols both satisfy a criterion ranked as first priority to the same extent, then a criterion ranked as second priority is considered.

Step 106 of generating an imaging protocol for the patient may comprise selecting the imaging protocol from a predefined set of imaging protocols. For example, step 106 may comprise determining which of the predefined set of imaging protocols will provide a reconstructed CBCT image of the patient in which the image quality of the reconstructed CBCT image of the patient meets the image quality objective for the image processing task.

Generating the imaging protocol (in step 106) by performing an optimization process may comprise applying an optimization procedure to the imaging protocol. The optimization procedure may seek to optimize an extent to which the image quality objective for the image processing task is satisfied by the imaging protocol by adjusting the at least one parameter in the imaging protocol. The image quality objective prioritises image quality for positions within the patient volume at which the RTP is sensitive to changes in anatomy (which may be determined based on the measure of sensitivity). The optimization may be subject to one or more constraints. The one or more constraints may comprise, for example, one or more of: a maximum total imaging dose of the imaging protocol; a maximum total image acquisition time of the imaging protocol; a maximum total reconstruction computation time of the imaging protocol; a physical constraint (e.g., of the system/hardware); a clinical constraint; and an image quality constraint (e.g., based on the determined measure of sensitivity). Any suitable optimisation algorithm may be used, e.g., gradient descent.

The optimization process may be performed by an optimiser. Inputs to the optimiser may include one or more of: a cost function, one or more optimisable parameters, one or more constraints, and a reference objective for the optimization. The reference objective for the optimization may comprise the image quality objective for the image processing task. The reference objective may comprise one or more further objectives for the imaging protocol. The one or more optimisable parameters may comprise the at least one parameter in the imaging protocol. The cost function may comprise terms representing the reference objective and/or the one or more constraints. For example, the cost function may comprise a mathematical expression representing an extent to which the imaging protocol meets the image quality objective for the image processing task. The cost function may comprise a difference between the expected image quality and a desired image quality according to the image quality objective. The image quality objective, the cost function and/or a constraint may be based on (e.g., expressed in terms of) the determined measure of sensitivity. The image quality obtained by an imaging protocol may be defined in relation to the performance criterion of the image processing task. The image quality obtained by an imaging protocol may be defined, for example, based on a signal to noise level, a directional signal to noise level, a gradient uncertainty, spatial resolution testing, and/or a Hounsfield Unit (HU) uncertainty. The image quality obtained by an imaging protocol may be used to determine a corresponding uncertainty in the results of the image processing task (e.g., image segmentation) and the impact this uncertainty has on the radiotherapy treatment plan quality. Thus, the cost function may comprise RTP quality metrics, e.g., dose-related metrics such as uncertainty in computed/expected treatment dose. As mentioned above, the one or more constraints may comprise one or more of: a maximum total imaging dose of the imaging protocol; a maximum total image acquisition time of the imaging protocol; a maximum total reconstruction computation time of the imaging protocol; a physical constraint (e.g., of the system/hardware); a clinical constraint; and an image quality constraint (e.g., based on the determined measure of sensitivity).

The output of the optimiser may comprise value(s) for the at least one parameter in the imaging protocol and optionally a result for the image quality objective. To determine the output, the optimiser may vary the optimisable parameters so as to minimise the cost function.

Step 106 of generating an imaging protocol for the patient may comprise adapting a reference imaging protocol, e.g., using an analytic or iterative optimization process. For example, the at least one parameter in the reference imaging protocol may be iteratively varied until the imaging protocol is optimized according to the image quality objective. In some embodiments, previously obtained CBCT images may be used to adjust the acquisition parameters. For example, the previously obtained CBCT images may be CBCT images from previous fractions of the radiotherapy treatment of the patient.

Using an ML process to generate 106 an imaging protocol for the patient, may comprise inputting into an ML model: (i) TP information; and (ii) the image quality objective for the image processing task. The ML model may be operable to output an imaging acquisition protocol for obtaining a reconstructed CBCT image of the patient that satisfies the image quality objective for the image processing task.

The method 100 may further comprise training an ML model to generate an imaging protocol for obtaining a reconstructed CBCT image of the patient volume by: (i) generating training data for training the ML model; and (ii) using the training data to update trainable parameters of the ML model. Generating training data for the ML model may comprise, for a plurality of patients: performing steps 102 to 106 of Figure 1 using an analytic process and/or an optimization process to generate the imaging acquisition protocol for the patient; and adding to a training data set the radiotherapy treatment plan information, the image quality objective for the image processing task, and the generated imaging acquisition protocol for CBCT imaging of the patient.

In some examples, generating training data for the ML model may comprise generating training data using a plan robustness evaluation, e.g., in which the at least one parameter in the imaging protocol is varied and the image processing task is performed on reconstructed CBCT images corresponding to each imaging protocol. The reconstructed CBCT images for each imaging protocol may be simulated from a (planning) CT. An image quality objective may be evaluated for the image processing task for each simulated CBCT image. For example, the image processing task may be organ segmentation, and the image quality objective may be expressed in terms of a performance criterion for the organ segmentation (e.g., uncertainty in the generated contours) and the total imaging dose. This data may be used to train a ML model.

The method 100 may further comprise initiating CBCT imaging of the patient according to the generated imaging protocol. For example, initiating CBCT imaging of the patient may comprise outputting the generated imaging protocol, e.g., to a processor and/or an imaging system.

In summary, the method 100 of Figure 1 provides an improved method for managing CBCT imaging. By considering the sensitivity of the RTP to changes in patient anatomy, an imaging protocol is generated that prioritises image quality where it is most needed for accurate image processing for the accurate delivery of the RTP (e.g., where it is most useful for clinicians). In doing so, the imaging dose delivered to the patient, and/or the imaging time for acquisition and/or reconstruction, may be reduced relative to existing methods whilst still enabling accurate image processing and RTP delivery.

**Figure 2** is a series of images illustrating a method according to some embodiments of the present disclosure. The method illustrated by Figure 2 may correspond to the method 100 of Figure 1.

Figure 2(a) depicts the radiation dose distribution to be delivered to a patient according to a volumetric modulated arc therapy (VMAT) radiotherapy treatment plan for the treatment of prostate cancer. The RTP was created on a planning CT scan across a three-dimensional patient volume. The image shown in Figure 2(a) is an axial dose distribution. The dose is depicted as a grayscale colour map with the darker regions representing higher dose. The solid lines delineate segments such as the PTV 201, which comprises the cancerous cells in the prostate to be treated, an OAR 202, which comprises the rectum, and the head of the right femur 203a and left femur 203b. The dose distribution depicted in Figure 2(a) is determined during a treatment planning process for the patient, i.e., when generating the radiotherapy treatment plan. The radiation dose distribution of Figure 2(a) may correspond to radiotherapy treatment plan information (as acquired in step 102 of the method 100 described with reference to Figure 1).

The 3D dose distribution depicted in Figure 2(a) is used to determine a measure of sensitivity of the RTP to changes in anatomy of the patient (corresponding to step 104 of Figure 1). To do so, the radiation dose gradient is calculated across a patient volume (i.e., the volume defined by the 3D dose distribution). This produces a vector field comprising a 3D dose gradient vector for each voxel in the patient volume. The high dose gradients at the skin-air interfaces (where the planned dose rapidly drops off) are excluded from the dose gradient so that the subsequent analysis is focused on dose changes within the body, not those at the skin-air interface.

The (cropped) dose gradient vector field is represented in Figure 2(b) as a grayscale colourmap showing the magnitude of the dose gradient across the axial plane. The darkest regions represent the highest dose gradient magnitude. In other words, the darkest regions are those with the greatest rate of change of dose (to be delivered to the patient according to the radiotherapy treatment plan). As can be seen in Figure 2(b), the region 204 comprising the highest gradient magnitude is located at the boundary between the prostate and the rectum, i.e., at the prostate-rectum interface. This is a key region of delineation for the radiotherapy treatment plan because the prostate is part of the PTV 201 whereas the rectum is part of an OAR 202. Therefore, the dose distribution changes sharply at the identified boundary and sufficient image quality is important for accurate delineation of this boundary for RT applications (e.g., for IGRT, ART, etc). A consequence of this sharp change in dose is that a small change in the patient anatomy (e.g., due to patient movement, weight gain, digestion, urinary processes, etc.) may be enough to prevent the radiotherapy treatment plan achieving its objectives. In other words, the RTP is more sensitive to changes in anatomy in regions where there is a high dose gradient. For example, a first objective of the RTP may comprise a minimum dose to be delivered to the PTV 201 for the effective treatment of the tumour. If a change in patient anatomy occurs between treatment planning and dose delivery, and that change causes a section of the PTV 201 to move across the identified boundary into the region of low dose intended for the OAR 202, then the dose delivered to the PTV 201 during treatment may fall below the minimum dose, thus violating the first RT treatment objective. A second objective of the RTP may comprise an upper limit on the dose that may be delivered to the OAR 202, e.g., to limit damage to the rectum during the treatment. If a change in patient anatomy causes a section of the OAR 202 to move into the region of high dose intended for the PTV 201, then the dose delivered to the OAR 202 may surpass the upper limit, thus violating the second RT treatment objective. Since the change in dose occurs abruptly at the identified boundary (i.e., the dose gradient in this region is high), the change in patient anatomy need only be small to violate the RTP. In other words, at the boundary, the RTP is highly sensitive to changes in anatomy of the patient, and the dose gradient provides a measure of this sensitivity. A threshold dose gradient value (and optionally a threshold dose) may be used as a sensitivity criterion to identify positions within the patient volume at which the RTP is sensitive to changes in anatomy. It will be appreciated that although Figure 2(b) depicts the dose gradient in two dimensions, the determined dose gradient is defined in three dimensions.

An imaging protocol for obtaining a reconstructed CBCT image of the patient volume is then generated. The reconstructed CBCT image may be used for IGRT and/or adaptive radiotherapy treatment. Thus, the reconstructed CBCT image may be used to ensure that the correct dose is delivered to the different regions of the patient so that the RT treatment objectives are met. The image quality in regions where the dose gradient is high is more important than the image quality in regions with lower dose gradient because the high dose gradient regions are more likely to violate the RTP due to changes in patient anatomy. Therefore, acquiring a precise understanding of the patient anatomy at positions within the patient volume at which the RTP is sensitive to changes in anatomy is more important than acquiring a precise understanding of the patient anatomy elsewhere in the reconstructed image. In this example, the generated imaging protocol is an image acquisition protocol. When generating the imaging protocol, prioritising image quality for positions within the patient volume at which the RTP is sensitive to changes in anatomy enables the imaging dose to be as low as possible whilst still acquiring sufficient data to generate a reconstructed CBCT image with enough detail for accurate IGRT and/or adaptive radiotherapy treatment. Generating the imaging protocol may correspond to step 106 of Figure 1.

In order to generate the imaging protocol, the following steps are performed:
(i) The dose gradient vector field (depicted in Figure 2(b)) is projected onto the plane of the detector for different CBCT projection angles (i.e., angular locations of the imaging source and imaging detector along the CBCT scan arc). The magnitude of the projected dose gradient vector is determined as a function of position on the detector plane.
(ii) A tapering filter is applied to focus more on projected dose gradients near the centre of the detector than those near the edge of the detector (since the regions sensitive to anatomy changes for which high quality images are beneficial are nearer to the centre of the detector). Other types of filters could be used to serve this purpose.
(iii) A mean intensity value is computed for each projection by averaging over the detector plane.
(iv) The mean intensity values (i.e., the mean projected dose gradient intensities) are normalised (i.e., scaled) subject to a certain criterion to provide a normalised mean intensity function. The criterion may correspond to the image quality objective described with reference to Figure 1 (the normalisation step used in this example will be described in more detail below). The normalised mean intensity function (a function of projection number) is plotted in Figure 2(c). In this example, the projections are equally spaced along the CBCT scan arc so that the projection number scales with projection angle on the scan arc.
(v) The normalised mean intensity function is used to modulate the imaging source tube current (mA) in a reference CBCT image acquisition protocol. This provides the image acquisition protocol.

Examples of the present disclosure may comprise performing any one or more of the above steps.

The dose gradient projection intensity plotted in Figure 2(c) reveals the projections for which the in-plane dose gradient is highest. These projections with the highest in-plane dose gradient are the projections capturing neighbouring position(s) in the patient for which there is a large change of dose requirement (indicating that the RTP is sensitive to changes in anatomy of the patient). Therefore, it is beneficial to prioritise the image quality for these projection angle(s) over the image quality for projection angles with lower in-plane dose gradient.

To achieve this, the normalised mean intensity plotted in Figure 2(c) is used as a modulation function for the imaging source current in the imaging protocol for a subsequent CBCT acquisition. In this case, the normalisation step mentioned above comprises normalising the mean dose gradient projection intensity function such that, when the normalised function is used to modulate the source current, an image quality objective is met. In this example, the image quality objective requires that the image quality of the reconstructed CBCT image is optimised for an image processing task (e.g., image segmentation, image registration) subject to a requirement that the total imaging dose delivered to the patient by the CBCT imaging protocol does not exceed a threshold value. The requirement to optimise image quality for image segmentation and/or image registration is satisfied by modulating the imaging source current in the imaging protocol with the normalised mean intensity of Figure 2(c) because it ensures that the projections that contribute most to imaging the sensitive regions in the patient (which are important for image segmentation/image registration) are imaged with a higher source current, thus improving image quality in these regions (e.g., improved CNR, SNR, spatial resolution, etc). Thus, the normalisation (i.e., scaling factor) for the mean dose gradient projection intensity function is determined such that, when the imaging source tube current in the reference CBCT image acquisition protocol is modulated by the normalised mean intensity, the total imaging dose delivered to the patient by the CBCT image acquisition protocol does not exceed the threshold value. Thus, the image quality objective is met.

To demonstrate the advantages of this method, CBCT image reconstructions of the patient volume were simulated for three different imaging protocols and the results are shown in Figures 2(d), 2(e) and 2(f) respectively.

Figure 2(d) shows an axial view of a simulated reconstructed CBCT image of the patient volume that was obtained using the reference CBCT imaging protocol (in which the imaging intensity has not been modulated according to the method described above). This reconstruction is referred to as the baseline. The imaged prostate-rectum interface 205 in the baseline reconstruction is pixelated and difficult to make out.

Figure 2(e) shows an axial view of a simulated reconstructed CBCT image of the patient volume that was obtained by increasing the imaging intensity in the reference CBCT imaging protocol by 100% for all projections. The prostate-rectum interface 206 is much sharper in this reconstruction compared to the baseline reconstruction, but the total imaging dose delivered to the patient is doubled.

Figure 2(f) shows an axial view of a simulated reconstructed CBCT image of the patient volume that was obtained by modulating the imaging intensity in the reference CBCT imaging protocol by the normalised mean intensity function of Figure 2(c). The image quality around the prostate-rectum interface 207 is again higher than in the baseline reconstruction (e.g., measured by signal to noise, uncertainty in interface position, intensity gradient steepness), but the total imaging dose is now only 38% higher than the baseline reconstruction. Thus, with the method disclosed herein, it is possible to accurately delineate the neighbouring PTV and OAR regions at the prostate-rectum interface whilst administering a lower total imaging dose to the patient (e.g., relative to the acquisition protocol of Figure 2(e)). Accurate delineation of these regions is important for detecting if the prostate-rectum interface has moved or changed between radiotherapy treatment planning and radiotherapy treatment delivery. If it has, the CBCT images may be used for patient realignment (e.g., IGRT) and/or adaptations to the radiotherapy treatment plan (e.g., ART).

In the example method depicted in Figure 2, an imaging acquisition protocol was generated by modulating the imaging intensity in a reference imaging acquisition protocol. However, it will be appreciated that one or more other parameters in the imaging acquisition protocol may (in addition or instead) be modulated, such as the sampling density of projections along a scan arc (i.e., the number of projections per gantry angular location) or gantry speed. For example, by increasing the sampling density (or decreasing gantry speed) for the projections with the highest projected dose gradient intensity, it would be possible to achieve an increase in image quality at positions in the patient at which the radiotherapy treatment plan is sensitive to changes in anatomy of the patient.

Furthermore, according to the method depicted in Figure 2, the imaging acquisition protocol was generated by starting from a reference imaging acquisition protocol (corresponding to the baseline reconstruction in Figure 2(d)) and modulating one of the imaging acquisition parameters (the imaging intensity). However, in alternative embodiments, the imaging acquisition protocol may be generated from scratch.

The imaging protocol generated in the method of Figure 2 was an imaging acquisition protocol. In alternative embodiments, the imaging protocol may be an imaging reconstruction protocol. For example, the image quality objective may be met by increasing the voxel density (e.g., decreasing voxel size) at positions within the patient volume at which the RTP is sensitive to changes in anatomy (e.g., by scaling the voxel density by the measure of sensitivity as a function of position in the patient).

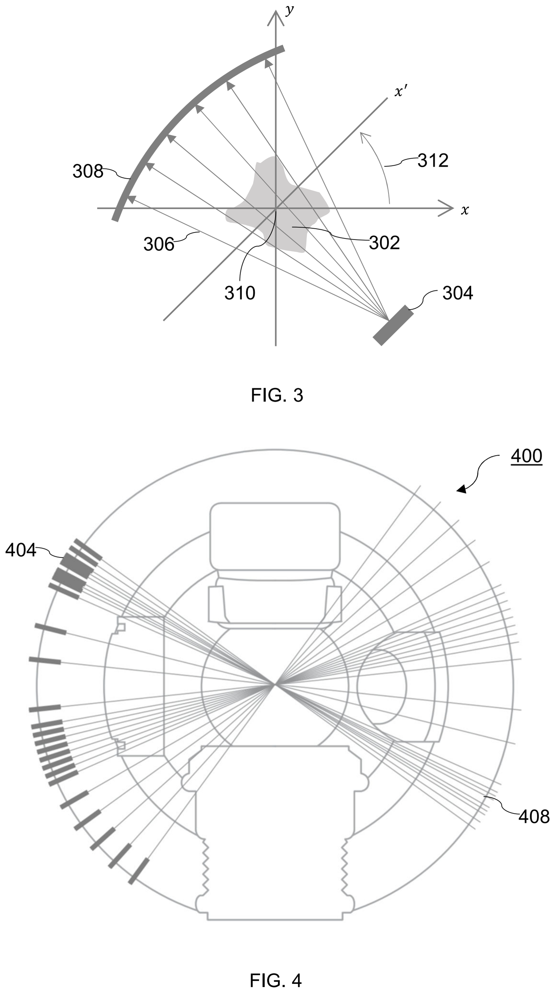

**Figure 3** illustrates the geometry of a typical cone beam projection acquisition setup, as may be used in image acquisition and image reconstruction techniques according to techniques of the present disclosure. For simplicity, only the x-y plane is illustrated; the skilled reader will appreciate that the beam of rays extends also into the z-axis, orthogonal to the x-y plane. In this example, the acquisition setup captures projection data of object 302 (which may, for example, be the patient). X-ray source 304 generates and emits X-rays 306 towards object 302. In CBCT, X-rays may be considered as a beam of rays, emitted from a point source. Detector (or detectors) 308 capture projections, which are sets of line integrals along paths that radiate from the source 304. Multiple projections of the image may be acquired from different angles by rotating the source 304 and detector 308 around the centre of the image 310. In the present example, the source 304 and detector 308 may be rotated arcuately along orbital path 312 (referred to herein as a scan arc or CBCT scan arc). In this way, the x-y plane may be rotated counterclockwise around the point of origin (or centre of the image 310) in a manner that keeps the mutual positional relationship between source 304 and detector 308 when passing through the orbital path 312. Other configurations of imaging systems are, of course, feasible; for instance, the source may be configured to rotate and a complete ring of detectors may be configured to capture projections, or there may be multiple sources arranged circumferentially around a complete ring of detectors. Further, the imaging system may rotate the source along a helical path, so as to capture projection data along the axis of the object 302 of interest (along the z-axis in the present example).

The attenuation of the intensity of the rays that pass through the object 302 may be measured by processing signals received from the detector 308. By making projective measurements at a series of different projection angles through the object 302, a sinogram may be constructed from the projection data, mapping the spatial dimension of the detector array to the projection angle dimension. The intensity attenuation resulting from a particular volume within the object will trace out a sine wave for the spatial dimension along the detector perpendicular to the rotation axis of the system. Volumes of the object farther from the centre of rotation correspond to sine waves with greater amplitudes than those corresponding to volumes nearer the centre of rotation. The phase of each sine wave in the sinogram corresponds to the relative angular positions with respect to the rotation axis. By performing an image reconstruction technique (such as an inverse Radon transform) on the projection data in the sinogram, one may reconstruct an image, where the reconstructed image corresponds to a cross-sectional slice or volume of the object 302. The detector 308 may comprise underlying detector elements (such as pixels or bins of pixels), rather than a single active region. The measured ray intensities may then be taken as a function of underlying intensity signals acquired at detector elements of the detector. This is advantageous in that one reduces the impact of random noise, which may plague an intensity (particularly a low intensity) measured at a single detector element. For example, the measured ray intensities may be an average (mean, mode or median) of underlying single detector element intensities.

**Figure 4** illustrates the geometry of a cone beam projection acquisition setup, as incorporated into a medical CT scanner 400. Radiation sources 404 emit beams of X-ray radiation, which may pass through the patient supported on a couch within the CT scanner (not depicted). Detectors 408 capture attenuated X-rays. The radiation sources 404 and detectors 408 may be configured to rotate within a gantry of the CT scanner, so as to acquire projective measurements at a series of different projection angles.

The CBCT projections in a CBCT image acquisition protocol are typically acquired at equidistant positions along a scan arc and with constant intensity and energy. However, as shown in Figure 4, the angular separation between measurement orientations need not be equal. For example, according to embodiments disclosed herein, a higher density of projection angles may be used to increase the image quality of the reconstructed CBCT image and to prioritise image quality for positions within the patient volume at which the RTP is sensitive to changes in anatomy. A set of densely sampled projections in a small sub arc may be combined with less densely sampled projections for the rest of the rotation to obtain improved image quality in some desired areas whilst minimising imaging dose. Information about optimising projection angle selection in Computed Tomography is provided in "Optimisation of projection angle selection in Computed Tomography" by Francien G. Bossema (Master Thesis Mathematics, University Leiden).

The image reconstruction method may account for flex within (or of) the gantry. For instance, in one implementation, the flex may be considered when determining where the isocentral ray hits the detector. In turn, the method may determine the intensity at the isocentral location through interpolation.

Referring back to the method of Figures 1 and 2, the imaging protocol generated in step 106 may comprise an imaging acquisition protocol for acquiring projection images using the set up described above in relation to Figures 3 and 4. The imaging protocol generated in step 106 may (in addition or instead) comprise an image reconstruction protocol for generating a reconstructed CBCT image based on projection images acquired using the set up described above in relation to Figures 3 and 4.

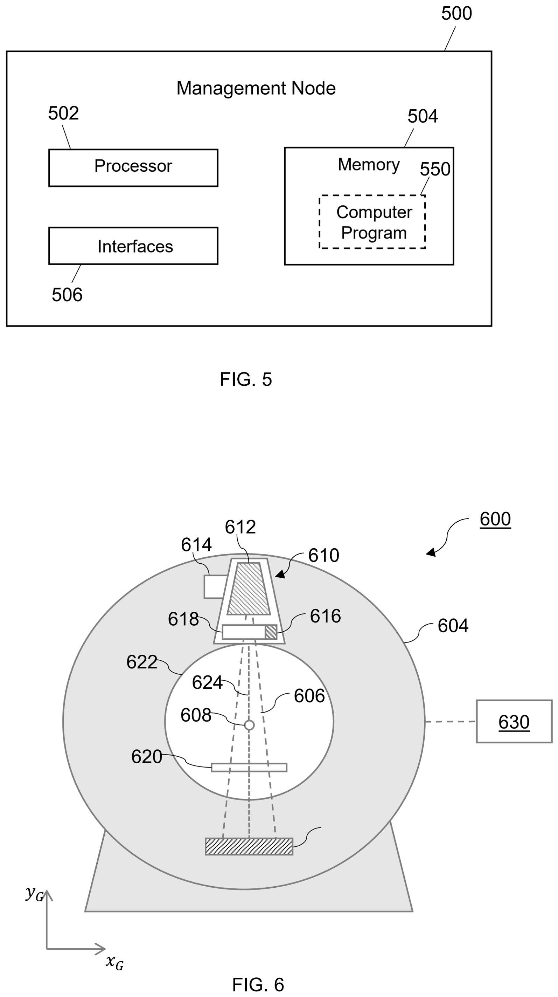

**Figure 5** is a block diagram illustrating an example management node 500 which may implement the method 100 illustrated in Figure 1, according to examples of the present disclosure. For example, the management node 500 may implement the method 100 of Figure 1 on receipt of suitable instructions from a computer program 550.

Referring to Figure 5 the management node 500 comprises a processor or processing circuitry 502, and may comprise a memory 504 and interfaces 506. The processing circuitry 502 is operable to perform some or all of the steps of the method 100 discussed above with reference to Figure 1. The memory 504 may contain instructions executable by the processing circuitry 502 such that the management node 500 is operable to perform some or all of the steps of the method 100 illustrated in Figure 1. The instructions may also include instructions for executing one or more telecommunications and/or data communications protocols. The instructions may be stored in the form of the computer program 550. In some examples, the processor or processing circuitry 502 may include one or more microprocessors or microcontrollers, as well as other digital hardware, which may include digital signal processors (DSPs), special-purpose digital logic, etc. The processor or processing circuitry 502 may be implemented by any type of integrated circuit, such as an Application Specific Integrated Circuit (ASIC), Field Programmable Gate Array (FPGA) etc. The processor or processing circuitry 502 may include a Graphics Processing Unit (GPU). The memory 504 may include one or several types of memory suitable for the processor, such as read-only memory (ROM), random-access memory, cache memory, flash memory devices, optical storage devices, solid state disk, hard disk drive, etc.

In some examples as discussed above, the management node may be incorporated into a radiotherapy treatment apparatus, and examples of the present disclosure also provide a radiotherapy treatment apparatus comprising the management node as discussed above. The radiotherapy treatment apparatus may be for image guided RT and/or ART.

**Figure 6** depicts a radiotherapy apparatus, suitable for performing radiotherapy treatment and/or image acquisition. The cross-section through radiotherapy apparatus 600 includes a radiation head 610 and a beam receiving apparatus (detector) 602, both of which are attached to a gantry 604. The radiation head 610 includes a radiation source 612, which emits a beam of radiation 606. The radiation head 610 also includes a beam shaping apparatus 618, which controls the size and shape of the radiation field associated with the beam.

The beam receiving apparatus 602 is configured to receive radiation emitted from the radiation head 610, for the purpose of absorbing and/or measuring the beam of radiation. In the view shown, the radiation head 610 and the beam receiving apparatus 602 are positioned diametrically opposed to one another.

The gantry 604 is rotatable, and supports the radiation head 610 and the beam receiving apparatus 602 such that they are rotatable around an axis of rotation 608, which may coincide with the patient longitudinal axis. The gantry provides rotation of the radiation head 610 and the beam receiving apparatus 602 in a plane perpendicular to the patient longitudinal axis (e.g., a sagittal plane). Three gantry directions *x_{G}, y_{G}, z_{G}* may be defined such that the *y_{G}* direction is perpendicular with the gantry axis of rotation. The *y_{G}* direction extends from a point on the gantry corresponding to the radiation head 610, towards the axis of rotation of the gantry. Therefore, from the patient frame of reference, the *y_{G}* direction rotates around as the gantry rotates.

The radiotherapy apparatus 600 also includes a support surface or couch 620 on which a subject (or patient) is supported during radiotherapy treatment or image acquisition. The radiation head 610 is configured to rotate around the axis of rotation 608 such that the radiation head 610 directs radiation towards the subject from various angles around the subject in order to spread out the radiation dose received by healthy tissue to a larger region of healthy tissue while building up a prescribed dose of radiation at a target region.

The radiotherapy apparatus 600 is configured to deliver a radiation beam towards a radiation isocentre, which is substantially located on the axis of rotation 608 at the centre of the gantry 604 regardless of the angle at which the radiation head 610 is placed.

The rotatable gantry 604 and radiation head 610 are dimensioned so as to allow a central bore 622 to exist. The central bore 622 provides an opening, sufficient to allow a subject to be positioned therethrough without the possibility of being incidentally contacted by the radiation head 610 or other mechanical components as the gantry rotates the radiation head 610 about the subject.

The radiation head 610 emits the radiation beam 606 along a beam axis 624 (or radiation axis or beam path), where the beam axis 624 is used to define the direction in which the radiation is emitted by the radiation head 610. The radiation beam 606 is incident on the beam receiving apparatus 602, which may include at least one of a beam stopper and a radiation detector. The beam receiving apparatus 602 is attached to the gantry 604 on a diametrically opposite side to the radiation head 610 to attenuate and/or detect a beam of radiation after the beam has passed through the subject.

The radiation beam axis 624 may be defined as, for example, a centre of the radiation beam 606 or a point of maximum intensity.

The beam shaping apparatus 618 delimits the spread of the radiation beam 606. The beam shaping apparatus 618 is configured to adjust the shape and/or size of a field of radiation produced by the radiation source. The beam shaping apparatus 618 does this by defining an aperture (also referred to as a window or an opening) of variable shape to collimate the radiation beam 606 to a chosen cross-sectional shape. In this example, the beam shaping apparatus 618 may be provided by a combination of a diaphragm and an MLC. Beam shaping apparatus 618 may also be referred to as a beam modifier.

The radiotherapy apparatus 600 may be configured to deliver both coplanar and non-coplanar (also referred to as tilted) modes of radiotherapy treatment. In coplanar treatment, radiation is emitted in a plane that is perpendicular to the axis of rotation of the radiation head 610. In non-coplanar treatment, radiation is emitted at an angle that is not perpendicular to the axis of rotation. In order to deliver coplanar and non-coplanar treatment, the radiation head 610 may move between at least two positions, one in which the radiation is emitted in a plane which is perpendicular to the axis of rotation (coplanar configuration) and one in which radiation is emitted in a plane which is not perpendicular to the axis of rotation (non-coplanar configuration).

In the coplanar configuration, the radiation head 610 is positioned to rotate about a rotation axis and in a first plane. In the non-coplanar configuration, the radiation head is tilted with respect to the first plane such that a field of radiation produced by the radiation head is directed at an oblique angle relative to the first plane and the rotation axis. **In** the non-coplanar configuration, the radiation head 610 is positioned to rotate in a respective second plane parallel to and displaced from the first plane. The radiation beam is emitted at an oblique angle with respect to the second plane, and therefore as the radiation head rotates the beam sweeps out a cone shape.

In one configuration, the beam receiving apparatus 602 may remain in the same place relative to the rotatable gantry when the radiotherapy apparatus is in both the coplanar and non-coplanar modes. Therefore, the beam receiving apparatus 602 is configured to rotate about the rotation axis in the same plane in both coplanar and non-coplanar modes. This may be the same plane as the plane in which the radiation head rotates. In alternative configurations, the beam receiving apparatus 601 may also rotate.

The beam shaping apparatus 610 is configured to reduce the spread of the field of radiation in the non-coplanar configuration in comparison to the coplanar configuration.

The radiotherapy apparatus 600 includes a controller 630, which is programmed to control the radiation source 612, beam receiving apparatus 606 and the gantry 602. Controller 630 may perform functions or operations such as radiotherapy treatment planning, treatment execution, image acquisition, image processing, motion tracking, motion management, and/or other tasks involved in a radiotherapy process.

Controller 630 is programmed to control various components of apparatus 600, such as gantry 604, radiation head 610, beam receiving apparatus 602, and support surface 620, so as to acquire projection data (i.e., projection images) suitable for image reconstruction.

Hardware components of controller 630 may include one or more computers (e.g., general purpose computers, workstations, servers, terminals, portable/mobile devices, etc.); processors (e.g., central processing units (CPUs), graphics processing units (GPUs), microprocessors, digital signal processors (DSPs), field programmable gate arrays (FPGAs), special-purpose or specially-designed processors, etc.); memory/storage devices such as a memory (e.g., read-only memories (ROMs), random access memories (RAMs), flash memories, hard drives, optical disks, solid-state drives (SSDs), etc.); input devices (e.g., keyboards, mice, touch screens, mics, buttons, knobs, trackballs, levers, handles, joysticks, etc.); output devices (e.g., displays, printers, speakers, vibration devices, etc.); circuitries; printed circuit boards (PCBs); or other suitable hardware. Software components of controller 630 may include operation device software, application software, etc.

The radiation head 610 may be connected to a head actuator 614, which is configured to actuate the radiation head 610, for example between a coplanar configuration and one or more non-coplanar configurations, or for example to actuate the radiation source 612 and/or detector 602 in response to detection of flex. This may involve translation and rotation of the radiation head 610 relative to the gantry. In some implementations, the head actuator may include a curved rail along which the radiation head 610 may be moved to adjust the position and angle of the radiation head 610. The controller 630 may control the configuration of the radiation head 630 via the head actuator 614.

The beam shaping apparatus 618 includes a shaping actuator 616. The shaping actuator is configured to control the position of one or more elements in the beam shaping apparatus 618 in order to shape the radiation beam 606. In some implementations, the beam shaping apparatus 616 includes an MLC, and the shaping actuator 616 includes means for actuating leaves of the MLC. The beam shaping apparatus 618 may further comprise a diaphragm, and the shaping actuator 616 may include means for actuating blocks of the diaphragm. The controller 630 may control the beam shaping apparatus 618 via the shaping actuator 616.

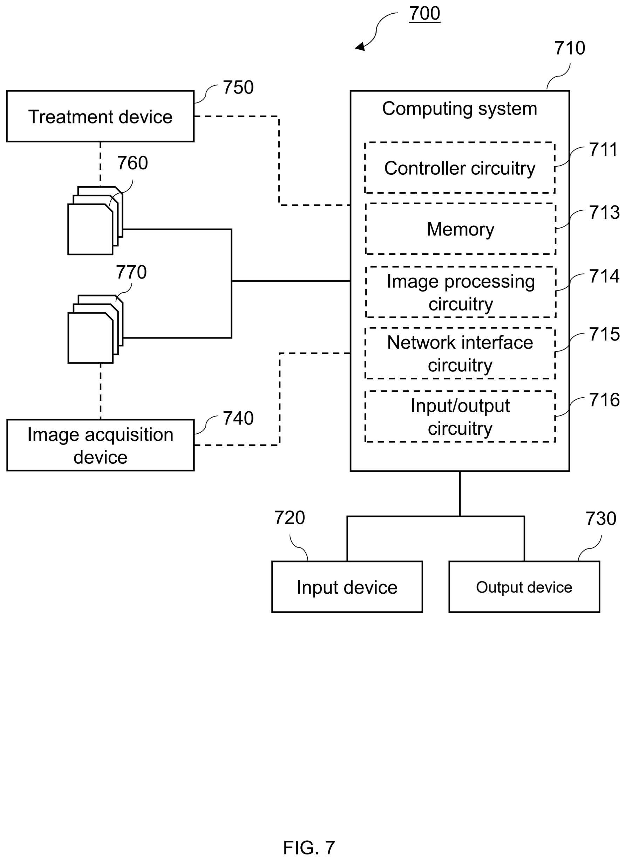

**Figure 7** is a block diagram of an implementation of a radiotherapy system 700, suitable for executing methods for managing CBCT imaging of a patient volume according to embodiments. The example radiotherapy system 700 comprises a computing system 710 within which a set of instructions, for causing the computing system 710 to perform the method (or steps thereof) discussed herein, may be executed. The computing system 710 may implement a CBCT imaging management system. The computing system 710 may also be referred to as a computer. In particular, the methods described herein may be implemented by a processor or controller circuitry 711 of the computing system 710.

The computing system 710 shall be taken to include any number or collection of machines, e.g., computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 710 includes controller circuitry 711 and a memory 713 (e.g., read-only memory (ROM), flash memory, dynamic random access memory (DRAM) such as synchronous DRAM (SDRAM) or Rambus DRAM (RDRAM), etc.). The memory 713 may comprise a static memory (e.g., flash memory, static random access memory (SRAM), etc.), and/or a secondary memory (e.g., a data storage device), which communicate with each other via a bus (not shown). Memory 713 may be used to store or buffer projection data until required for image processing.

Controller circuitry 711 represents one or more general-purpose processors such as a microprocessor, central processing unit, accelerated processing units, or the like. More particularly, the controller circuitry 711 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 711 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 711 is configured to execute the processing logic for performing the operations and steps discussed herein.

The computing system 710 may further include a network interface circuitry 715. The computing system 710 may be communicatively coupled to an input device 720 and/or an output device 730, via input/output circuitry 716. In some implementations, the input device 720 and/or the output device 730 may be elements of the computing system 710. The input device 720 may include an alphanumeric input device (e.g., a keyboard or touchscreen), a cursor control device (e.g., a mouse or touchscreen), an audio device such as a microphone, and/or a haptic input device. The output device 730 may include an audio device such as a speaker, a video display unit (e.g., a liquid crystal display (LCD) or a cathode ray tube (CRT)), and/or a haptic output device. In some implementations, the input device 720 and the output device 730 may be provided as a single device, or as separate devices.

In some implementations, the computing system 710 may comprise image processing circuitry 714. Image processing circuitry 714 may be configured to process image data 770 (e.g., images, imaging data, projections, projection data), such as medical images obtained from one or more imaging data sources, a treatment device 750 and/or an image acquisition device 740. Image processing circuitry 714 may be configured to process, or pre-process, image data 770. For example, image processing circuitry 714 may convert received image data into a particular format, size, resolution or the like. Image processing circuitry 714 may be configured to perform image reconstruction. In some implementations, image processing circuitry 714 may be combined with controller circuitry 711.

In some implementations, the radiotherapy system 700 may further comprise an image acquisition device 740 and/or a treatment device 750. The image acquisition device 740 and the treatment device 750 may be provided as a single device. In some implementations, treatment device 750 is configured to perform imaging, for example in addition to providing treatment and/or during treatment.

Image acquisition device 740 may be configured to perform CBCT. Image acquisition device 740 may be configured to perform positron emission tomography (PET), computed tomography, magnetic resonance imaging (MRI), single positron emission computed tomography (SPECT), X-ray, and the like.

Image acquisition device 740 may be configured to output image data 770, which may be accessed by computing system 710. Treatment device 750 may be configured to output treatment data 760, which may be accessed by computing system 710. Treatment data 760 may be obtained from an internal data source (e.g., from memory 713) or from an external data source, such as treatment device 750 or an external database.

The various methods described above may be implemented by a computer program. The computer program may include computer code (e.g., instructions) arranged to instruct a computer to perform the functions of one or more of the various methods described above. For example, the steps of the methods described in relation to Figure 1 and/or Figure 2 may be performed by the computer code. The steps of the methods described above may be performed in any suitable order. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions may also reside, completely or at least partially, within the memory 913 and/or within the controller circuitry 911 during execution thereof by the computing system 910, the memory 913 and the controller circuitry 911 also constituting computer-readable storage media.

In an implementation, the modules, components and other features described herein may be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components may be implemented as firmware or functional circuitry within hardware devices. Further, the modules and components may be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining", "identifying", "obtaining", "accessing", or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It should be noted that the above-mentioned examples illustrate rather than limit the disclosure, and that those skilled in the art will be able to design many alternative embodiments without departing from the scope of the appended claims or numbered embodiments. The word "comprising" does not exclude the presence of elements or steps other than those listed in a claim or embodiment, "a" or "an" does not exclude a plurality, and a single processor or other unit may fulfil the functions of several units recited in the claims or numbered embodiments. Any reference signs in the claims or numbered embodiments shall not be construed so as to limit their scope.

The following numbered statements set out embodiments of the disclosure:
1. A computer-implemented method for managing Cone Beam Computed Tomography, CBCT, imaging of a patient, the method comprising:
   acquiring radiotherapy treatment plan, RTP, information for a RTP, wherein the RTP is for delivering a radiation dose distribution to the patient so as to respect at least one radiotherapy treatment objective;
   determining, based on the acquired RTP information, a measure of sensitivity of the RTP to changes in anatomy of the patient, wherein the measure of sensitivity is a function of position in the patient or a function of radiotherapy beam direction in the RTP; and
   generating, based on the determined measure of sensitivity, an imaging protocol for obtaining a reconstructed CBCT image of a volume of the patient, wherein the imaging protocol comprises values for at least one CBCT imaging acquisition parameter and/or at least one CBCT imaging reconstruction parameter, by:
      setting a value of at least one parameter in the imaging protocol such that image quality of the reconstructed CBCT image of the patient volume meets an image quality objective for an image processing task, wherein the image quality objective prioritises image quality for positions within the patient volume at which the RTP is sensitive to changes in anatomy.
2. A method of embodiment 1, wherein the measure of sensitivity represents a degree to which the RTP would require adaptation as a consequence of changes to the patient anatomy in order to respect one or more of the at least one radiotherapy treatment objective.
3. A method of embodiment 1 or 2, wherein the measure of sensitivity comprises one or more of:
   a gradient of the radiation dose distribution as a function of position in the patient;
   a scale of patient anatomy change that would necessitate adaptation of the RTP in order to respect one or more of the at least one radiotherapy treatment objective as a function of position in the patient;
   the radiation dose distribution as a function of position in the patient;
   radiation to be delivered to the patient as a function of radiotherapy beam direction in the RTP; and
   monitor units, MU, to be delivered to the patient as a function of radiotherapy beam direction in the RTP.
4. A method of any one of the preceding embodiments, wherein positions within the patient volume at which the RTP is sensitive to changes in anatomy comprise positions at which the measure of sensitivity satisfies a sensitivity criterion.
5. A method of embodiment 4, wherein the sensitivity criterion comprises one or more of: a threshold radiation dose gradient; a reference scale anatomy change that would necessitate adaptation of the RTP in order to respect one or more of the at least one radiotherapy treatment objective; a threshold radiation dose; a threshold level of radiation to be delivered to the patient; and a threshold number of monitor units to be delivered to the patient.
6. A method of any one of the preceding embodiments, wherein changes in anatomy of the patient comprise one or more of:
   soft tissue movement;
   soft tissue shape changes; and
   overall patient anatomy changes.
7. A method of any one of the preceding embodiments, wherein the image quality objective seeks to balance a first requirement to optimize, for the image processing task, the image quality of the reconstructed CBCT image, with one or both of: a second requirement to minimise total imaging radiation dose delivered to the patient during CBCT image acquisition, and a third requirement to minimise total computation time for image reconstruction.
8. A method of any one of the preceding embodiments, wherein the image quality objective comprises a performance criterion for the image processing task performed on the reconstructed CBCT image.
9. A method of any one of the preceding embodiments, wherein the determined measure of sensitivity is a function of position in the patient, and setting a value of at least one parameter in the imaging protocol comprises:
   projecting the determined measure of sensitivity according to a geometry of the CBCT imaging of the patient to obtain a projected measure of sensitivity as a function of CBCT projection angle; and
   determining a value for at least one CBCT imaging acquisition parameter as a function of CBCT projection angle based on the projected measure of sensitivity and the image quality objective.
10. A method of any one of the preceding embodiments, wherein the determined measure of sensitivity is a function of position in the patient, and setting a value of at least one parameter in the imaging protocol comprises:
   determining a value for at least one CBCT imaging reconstruction parameter as a function of position in the patient volume based on the determined measure of sensitivity and the image quality objective.
11. A method of any one of the preceding embodiments, wherein the at least one CBCT imaging acquisition parameter comprises at least one of:
   imaging radiation source intensity for one or more projections;
   imaging source current for one or more projections;
   exposure time for one or more projections;
   imaging radiation source energy for one or more projections;
   gantry rotation speed for one or more projections;
   gantry angular range for one or more projections;
   rotation plane for one or more projections;
   isocentre for one or more projections;
   image projection angle for one or more projections;
   number of projections per CBCT acquisition angular range;
   imaging beam shape for one or more projections;
   detector offset for one or more projections;
   detector sensitivity for one or more projections; and
   detector gain for one or more projections.
12. A method of any one of the preceding embodiments, wherein the at least one CBCT imaging reconstruction parameter comprises at least one of:
   voxel size for one or more regions of the reconstructed CBCT image;
   number of voxels for one or more regions of the reconstructed CBCT image;
   voxel density for one or more regions of the reconstructed CBCT image;
   voxel distribution for one or more regions of the reconstructed CBCT image;
   one or more regularisation terms;
   number of iterations; and
   a stopping criterion.
13. A method of any one of the preceding embodiments, wherein generating an imaging protocol comprises:
   generating a plurality of candidate imaging protocols for the patient; and
   selecting one of the plurality of candidate imaging protocols for implementation.
14. A method of embodiment 13, wherein selecting one of the plurality of candidate imaging protocols for implementation comprises selecting according to a selection criterion comprising at least one of:
   total imaging radiation dose according to the candidate imaging protocols;
   image quality of the reconstructed CBCT image according to the candidate imaging protocols;
   total imaging and/or reconstruction time according to the candidate imaging protocols; and
   an extent to which the image quality objective is met by the candidate imaging protocols.
15. A method of any one of the preceding embodiments, wherein:
   determining a measure of sensitivity of the RTP to changes in anatomy of the patient; and/or
   generating an imaging protocol for obtaining a reconstructed CBCT image of a volume of the patient;
   comprise performing at least one of:
      an analytic process;
      an optimization process; and/or
      a Machine Learning, ML, process.
16. A method of embodiment 15, wherein performing an optimization process to generate an imaging protocol for the patient comprises:
   applying an optimization procedure to the imaging protocol, wherein the optimization procedure seeks to optimize an extent to which the image quality objective for the image processing task is satisfied by the imaging protocol by adjusting the at least one parameter in the imaging protocol.
17. A method of embodiment 15 or 16, wherein using an ML process to generate an imaging protocol for obtaining a reconstructed CBCT image of the patient volume;
   comprises:
   inputting to an ML model the RTP information and the image quality objective for the image processing task, wherein the ML model is operable to output the imaging protocol for obtaining a reconstructed CBCT image of the patient that satisfies the image quality objective for the imaging processing task.
18. A method of any one of embodiments 15 to 17, further comprising:
   training an ML model to generate an imaging protocol for the patient by:
      generating training data for training the ML model; and
      using the training data to update trainable parameters of the ML model;
   wherein generating training data for the ML model comprises, for a plurality of patients:
      performing the steps of embodiment 1 using an analytic process and/or an optimization process to generate the imaging protocol for the patient; and
      adding to a training data set the RTP information and the generated imaging protocol for obtaining a reconstructed CBCT image of the patient.
19. A method of any one of the preceding embodiments, wherein determining the measure of sensitivity of the RTP to changes in the anatomy of the patient comprises:
   obtaining a prediction of patient movement during the CBCT imaging of the patient and/or during radiotherapy treatment of the patient; and
   evaluating sensitivity of the RTP to the predicted patient movement.
20. A method of any one of the preceding embodiments, wherein the image processing task comprises one or more of: image reconstruction; image segmentation; image contouring; target region identification; organ at risk identification; image registration; and motion tracking.
21. A method of any one of the preceding embodiments, wherein the RTP information comprises one or more of: the RTP for the patient; the radiation dose distribution for the patient; a gradient of the radiation dose distribution for the patient; a radiation fluence map; a Dose Volume Histogram, DVH, for the patient; monitor units, MU, as a function of radiotherapy beam direction according to the RTP; an image of the patient; a segmented image of the patient; a patient model; the at least one radiotherapy treatment objective; a configuration of a radiotherapy system for delivering the radiation dose distribution; one or more parameters for configuring the radiotherapy system; and a delivery sequence for the radiotherapy system for delivering the radiation dose distribution.
22. A management node for managing Cone Beam Computed Tomography, CBCT, imaging of a patient, the management node comprising processing circuitry configured to cause the management node to:
   acquire radiotherapy treatment plan, RTP, information for a RTP, wherein the RTP is for delivering a radiation dose distribution to the patient so as to respect at least one radiotherapy treatment objective
   determine, based on the acquired RTP information, a measure of sensitivity of the RTP to changes in anatomy of the patient, wherein the measure of sensitivity is a function of position in the patient or a function of radiotherapy beam direction in the RTP; and
   generate, based on the determined measure of sensitivity, an imaging protocol for obtaining a reconstructed CBCT image of a volume of the patient, wherein the imaging protocol comprises values for at least one CBCT imaging acquisition parameter and/or at least one CBCT imaging reconstruction parameter, by:
      setting a value of at least one parameter in the imaging protocol such that image quality of the reconstructed CBCT image of the patient volume meets an image quality objective for an image processing task, wherein the image quality objective prioritises image quality for positions within the patient volume at which the RTP is sensitive to changes in anatomy.
23. A management node of embodiment 22, wherein the processing circuitry is further configured to cause the management node to carry out a method according to any one of embodiments 2 to 21.
24. A radiotherapy treatment apparatus comprising a management node of embodiment 22 or 23.
25. A computer program product comprising a computer readable medium, the computer readable medium having computer readable code embodied therein, the computer readable code being configured such that, on execution by a suitable computer or processor, the computer or processor is caused to perform a method of any one of embodiments 1 to 21.

## Claims

1. A computer-implemented method for managing Cone Beam Computed Tomography, CBCT, imaging of a patient, the method comprising:
acquiring radiotherapy treatment plan, RTP, information for a RTP, wherein the RTP is for delivering a radiation dose distribution to the patient so as to respect at least one radiotherapy treatment objective;
determining, based on the acquired RTP information, a measure of sensitivity of the RTP to changes in anatomy of the patient, wherein the measure of sensitivity is a function of position in the patient or a function of radiotherapy beam direction in the RTP; and
generating, based on the determined measure of sensitivity, an imaging protocol for obtaining a reconstructed CBCT image of a volume of the patient, wherein the imaging protocol comprises values for at least one CBCT imaging acquisition parameter and/or at least one CBCT imaging reconstruction parameter, by:
setting a value of at least one parameter in the imaging protocol such that image quality of the reconstructed CBCT image of the patient volume meets an image quality objective for an image processing task, wherein the image quality objective prioritises image quality for positions within the patient volume at which the RTP is sensitive to changes in anatomy.

2. A method as claimed in claim 1, wherein the measure of sensitivity represents a degree to which the RTP would require adaptation as a consequence of changes to the patient anatomy in order to respect one or more of the at least one radiotherapy treatment objective.

3. A method as claimed in claim 1 or 2, wherein the measure of sensitivity comprises one or more of:
a gradient of the radiation dose distribution as a function of position in the patient;
a scale of patient anatomy change that would necessitate adaptation of the RTP in order to respect one or more of the at least one radiotherapy treatment objective as a function of position in the patient;
the radiation dose distribution as a function of position in the patient;
radiation to be delivered to the patient as a function of radiotherapy beam direction in the RTP; and
monitor units, MU, to be delivered to the patient as a function of radiotherapy beam direction in the RTP.

4. A method as claimed in any one of the preceding claims, wherein the image quality objective seeks to balance a first requirement to optimize, for the image processing task, the image quality of the reconstructed CBCT image, with one or both of: a second requirement to minimise total imaging radiation dose delivered to the patient during CBCT image acquisition, and a third requirement to minimise total computation time for image reconstruction.

5. A method as claimed in any one of the preceding claims, wherein the determined measure of sensitivity is a function of position in the patient, and setting a value of at least one parameter in the imaging protocol comprises:
projecting the determined measure of sensitivity according to a geometry of the CBCT imaging of the patient to obtain a projected measure of sensitivity as a function of CBCT projection angle; and
determining a value for at least one CBCT imaging acquisition parameter as a function of CBCT projection angle based on the projected measure of sensitivity and the image quality objective.

6. A method as claimed in any one of the preceding claims, wherein the determined measure of sensitivity is a function of position in the patient, and setting a value of at least one parameter in the imaging protocol comprises:
determining a value for at least one CBCT imaging reconstruction parameter as a function of position in the patient volume based on the determined measure of sensitivity and the image quality objective.

7. A method as claimed in any one of the preceding claims, wherein the at least one CBCT imaging acquisition parameter comprises at least one of:
imaging radiation source intensity for one or more projections;
imaging source current for one or more projections;
exposure time for one or more projections;
imaging radiation source energy for one or more projections;
gantry rotation speed for one or more projections;
gantry angular range for one or more projections;
rotation plane for one or more projections;
isocentre for one or more projections;
image projection angle for one or more projections;
number of projections per CBCT acquisition angular range;
imaging beam shape for one or more projections;
detector offset for one or more projections;
detector sensitivity for one or more projections; and
detector gain for one or more projections.

8. A method as claimed in any one of the preceding claims, wherein the at least one CBCT imaging reconstruction parameter comprises at least one of:
voxel size for one or more regions of the reconstructed CBCT image;
number of voxels for one or more regions of the reconstructed CBCT image;
voxel density for one or more regions of the reconstructed CBCT image;
voxel distribution for one or more regions of the reconstructed CBCT image;
one or more regularisation terms;
number of iterations; and
a stopping criterion.

9. A method as claimed in any one of the preceding claims, wherein:
determining a measure of sensitivity of the RTP to changes in anatomy of the patient; and/or
generating an imaging protocol for obtaining a reconstructed CBCT image of a volume of the patient;
comprise performing at least one of:
an analytic process;
an optimization process; and/or
a Machine Learning, ML, process.

10. A method as claimed in any one of the preceding claims, wherein the image processing task comprises one or more of: image reconstruction; image segmentation; image contouring; target region identification; organ at risk identification; image registration; and motion tracking.

11. A method as claimed in any one of the preceding claims, wherein the RTP information comprises one or more of: the RTP for the patient; the radiation dose distribution for the patient; a gradient of the radiation dose distribution for the patient; a radiation fluence map; a Dose Volume Histogram, DVH, for the patient; monitor units, MU, as a function of radiotherapy beam direction according to the RTP; an image of the patient; a segmented image of the patient; a patient model; the at least one radiotherapy treatment objective; a configuration of a radiotherapy system for delivering the radiation dose distribution; one or more parameters for configuring the radiotherapy system; and a delivery sequence for the radiotherapy system for delivering the radiation dose distribution.

12. A management node for managing Cone Beam Computed Tomography, CBCT, imaging of a patient, the management node comprising processing circuitry configured to cause the management node to:
acquire radiotherapy treatment plan, RTP, information for a RTP, wherein the RTP is for delivering a radiation dose distribution to the patient so as to respect at least one radiotherapy treatment objective
determine, based on the acquired RTP information, a measure of sensitivity of the RTP to changes in anatomy of the patient, wherein the measure of sensitivity is a function of position in the patient or a function of radiotherapy beam direction in the RTP; and
generate, based on the determined measure of sensitivity, an imaging protocol for obtaining a reconstructed CBCT image of a volume of the patient, wherein the imaging protocol comprises values for at least one CBCT imaging acquisition parameter and/or at least one CBCT imaging reconstruction parameter, by:
setting a value of at least one parameter in the imaging protocol such that image quality of the reconstructed CBCT image of the patient volume meets an image quality objective for an image processing task, wherein the image quality objective prioritises image quality for positions within the patient volume at which the RTP is sensitive to changes in anatomy.

13. A management node as claimed in claim 12, wherein the processing circuitry is further configured to cause the management node to carry out a method according to any one of claims 2 to 11.

14. A radiotherapy treatment apparatus comprising a management node as claimed in claim 12 or 13.

15. A computer program product comprising a computer readable medium, the computer readable medium having computer readable code embodied therein, the computer readable code being configured such that, on execution by a suitable computer or processor, the computer or processor is caused to perform a method as claimed in any one of claims 1 to 11.
